# EUROPEAN PATENT SPECIFICATION

(11) **EP 0 951 284 B1**
(45) Date of publication and mention of the grant of the patent: **08.10.2003**
(21) Application number: 97952645.6
(22) Date of filing: 18.12.1997
(51) Int. Cl.: A61K 31/27, C07C 333/04, C07C 333/06, C07C 333/08, C07C 271/42, C07C 271/44, C07C 271/56, C07C 271/58

(54) **PHENYLETHYLAMINE DERIVATIVES**
PHENYLETHYLAMIN-DERIVATE
DERIVES DE PHENYLETHYLAMINE

(30) Priority: 18.12.1996 IL 11985296; 24.03.1997 IL 12050997
(43) Date of publication of application: 27.10.1999
(73) Proprietor: TEVA PHARMACEUTICAL INDUSTRIES, LTD., Jerusalem 91010 (IL); YISSUM RESEARCH DEVELOPMENT COMPANY OF THE HEBREW UNIVERSITY OF JERUSALEM, Jerusalem 92 182 (IL); Technion Research & Development Foundation Limited, Haifa 32000 (IL)
(72) Inventor: CHOREV, Michael, Jerusalem 93812 (IL); GOREN, Tamar, Rehovot 76310 (IL); HERZIG, Yacov, Ra'ananna 43266 (IL); STERLING, Jeffrey, Jerusalem 97275 (IL); WEINSTOCK-ROSIN, Marta, Jerusalem, 93222 (IL); YOUDIM, Moussa, B., H., Haifa, 33000 (IL)
(74) Representative: VOSSIUS & PARTNER
(86) International application number: US9723897
(87) International publication number: WO98026775

(56) References cited:
- US-A- 3 507 962
- US-A- 3 513 240
- US-A- 4 948 807
- US-A- 5 602 176
- DATABASE CAPLUS ON STN(R), CHEMICAL ABSTRACTS SERVICE, (Columbus, Ohio, USA), Accession Number 1996:340192, TERNI et al., "Preparation of (Aminoalkyl)Phenyl Morpholinoalkylcarbamates and Analogs as Cholinesterase Inhibitors", XP002909802 & WO,A,96 02524 (01 February 1996).
- DATABASE CAPLUS ON STN(R), CHEMICAL ABSTRACTS SERVICE, (Columbus, Ohio, USA), Accession Number 1980:180807, HORI et al., "N-Containing Diphenylethylamine Derivatives and Acid Adducts", XP002909803 & JP,A,54 132 559 (15 October 1979).

## Description

The present invention relates to compounds, pharmaceutical compositions containing said compounds and their use in the treatment of various CNS disorders.

Dementia may take several forms including static dementia, Alzheimer's-type dementia, senile dementia, presenile dementia and progressive dementia. One of the common pathological features of several types of dementia is the lack of the neurotransmitter acetylcholine. This has led to the development of acetylcholine esterase inhibitors for use in the treatment of dementia's such as the compound tacrine.

Recently, compounds that in addition to inhibiting acetylcholine esterase, have inhibitory activity against monoamine oxidase type A (MAO-A) have been developed. The perceived benefit of having the anti-MAO-A activity is stated to be an anti-depressant effect (European Patent Applications Publication Nos. 614,888 and 664,291). Fink et al., (Bioorg & Med Chem Letts (1996) 6 625-630) also disclose compounds having both acetylcholine esterase and monoamine oxidase inhibitory moieties.

International Patent Application Publication No. WO96/02524 relates to alkylamino substituted phenylcarbamate derivatives having acetylcholinesterase inhibitory activity and their use in the treatment of Alzheimer's Disease.

The present invention relates to compounds of formula I having the following formula: 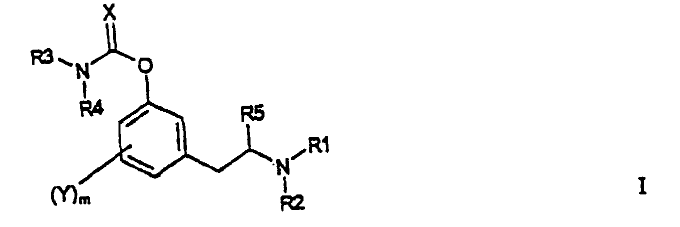 wherein m is from 0-4; X is O or S; Y is halogeno; R₁ is hydrogen or C₁₋₄ alkyl; R ₂ is hydrogen, C₁₋₄ alkyl or optionally substituted propargyl; R₃ and R₄ are each independently hydrogen, C₁₋₈ alkyl, C₆₋₁₂ aryl, C₆₋₁₂ cycloalkyl or C₆₋₁₂ aralkyl; and R₅ is hydrogen or C₁₋₄ alkyl, with the proviso that when X is O, R₂ is optionally substituted propargyl.

As used hereinafter, references to the compounds of formula I include all compounds of formula I without the final proviso.

The invention relates to the compounds themselves, pharmaceutical compositions containing said compounds and their use in the treatment of depression, Attention Deficit Disorder (ADD), Attention Deficit and Hyperactivity Disorder (ADHD), Tourette's Syndrome, Alzheimer's Disease and other dementias such as senile dementia, dementia of the Parkinson's type, vascular dementia and Lewy body dementia.

A further aspect of the present invention relates to the use of the compounds of formula I wherein m is from 0-4; X is O or S; Y is halogeno; R₁ is hydrogen or C₁₋₄ alkyl; R₂ is hydrogen, C₁₋₄ alkyl or optionally substituted propargyl; R₃ and R₄ are each independently hydrogen, C₁₋₈ alkyl, C₆₋₁₂ aryl, C₆₋₁₂ cycloalkyl or C₆₋₁₂ aralkyl; and R₅ is hydrogen or C₁₋₄ alkyl, in the treatment of neurotrauma, memory disorder or depression.

As used herein the term "neurotrauma" includes, but is not limited to, damage caused to the nervous system (both central and peripheral) by virtue of ischemic damage such as stroke, hypoxia or anoxia, neurodegenerative diseases, Parkinson's Disease, Alzheimer's Disease, Huntington's Disease, neurotoxic injury, head trauma injury, spinal trauma injury, peripheral neuropathy and any form of nerve damage.

The present invention relates to the racemic compounds themselves and optically active isomers thereof.

The present invention is directed to compounds having the following formula: 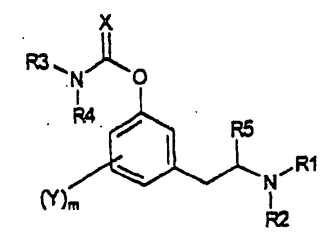 wherein m is from 0-4;
X is O or S;
Y is halogeno;
R₁ is hydrogen or C₁₋₄ alkyl;
R₂ is hydrogen, C₁₋₄ alkyl or optionally substituted propargyl; and R₃ and R, are each independently hydrogen, C₁₋₈ alkyl, C₆₋₁₂ aryl, C₆₋₁₂ cycloalkyl or C₆₋₁₂ aralkyl, R₅ is hydrogen or C₁₋₄ alkyl; and pharmaceutically acceptable salts thereof, provided that when X is O, R₂ is optionally substituted propargyl.

In an embodiment of the present invention, X is O. In a further embodiment of the present invention, X is S.

In an embodiment of the present invention, the compound is selected from the group consisting of: (rac)-3-(N-methyl,N-propyl-carbamyloxy)-α-methyl-N'-propargyl phenethylamine HCl; (rac)-3-(N,N-dimethyl-carbamyloxy) -α-methyl-N'-methyl,N'-propargyl phenethylamine HCl; (rac)-3-(N-methyl,N-hexyl-carbamyloxy)-α-methyl-N'-methyl, N'-propargyl phenethylamine mesylate; (rac)-3-(N-methyl,N-cyclohexyl-carbamyloxy)-α-methyl-N'-methyl, N'-propargyl phenethylamine HCl; and (S)-3-(N-methyl, N-hexyl-carbamyloxy)-α-methyl-N'-methyl,N'-propargyl phenethylamine ethanesulfonate.

A first object of the present invention relates to the compounds of formula I and their use in the treatment of Alzheimer's Disease and related dementias.

In an embodiment of the present invention R₁ is hydrogen, methyl or ethyl. When R₂ is propargyl then it may optionally be substituted. Such substitution is preferably on the methylene group (see R₆ in Scheme I) and is selected from the group consisting of C₁₋₄ alkyl.

According to the present invention, "halogeno" is used to refer to fluoro, chloro, bromo or iodo.

In an embodiment of the present invention, when m is greater than 1, each Y may be the same or different.

In a further embodiment of the present invention, at least one of R₃ and R₄ is methyl and the other is hydrogen, methyl, ethyl propyl, hexyl or cyclohexyl. In an additional embodiment of the present invention, R₅ is hydrogen or methyl. In a further embodiment, m=0; x=0; R₁ is methyl, R₂ is propargyl; R₃ is methyl; R₄ is ethyl; and R₆ is methyl.

The subject invention further provides pharmaceutically acceptable salts of the compounds of formula I. Examples of pharmaceutically acceptable salts include, but are not limited to, esylate salts, mesylate salts, maleate salts, fumarate salts, tartrate salts, hydrochloride salts, hydrdbromide salts, p-toluenesulfonate salts, benzoate salts, acetate salts, phosphate salts and sulfate salts.

The subject invention further provides a pharmaceutical composition which comprises a therapeutically effective amount of a compound of formula I or a pharmaceutically acceptable salt thereof and a pharmaceutically acceptable carrier. The "therapeutically effective amount" of a compound of formula I or a pharmaceutically acceptable salt thereof may be determined according to methods well known to those skilled in the art.

The compositions of the present invention may be prepared as medicaments to be administered orally, parenterally, rectally, or transdermally.

Suitable forms for oral administration include tablets, compressed or coated pills, dragees, sachets, hard or soft gelatin capsules, sublingual tablets, syrups and suspensions. In one embodiment, the pharmaceutically acceptable carrier is a solid and the pharmaceutical composition is a tablet.

The therapeutically effective amount may be an amount from about 0.5mg to about 2000 mg, preferably, from about 1mg to about 1000mg.

In an alternative embodiment, the pharmaceutically acceptable carrier is a liquid and the pharmaceutical composition is an injectable solution. The therapeutically effective amount may be an amount from about 0.5mg to about 2000mg, preferably from 1mg to about 1000mg. The volume administered may be an amount between 0.5 and 10ml.

In a further alternative embodiment, the carrier is a gel and the pharmaceutical composition is a suppository. For parenteral administration the invention provides ampoules or vials that include an aqueous or non-aqueous solution or emulsion. For rectal administration there are provided suppositories with hydrophilic or hydrophobic vehicles. For topical application as ointments and transdermal delivery there are provided suitable delivery systems as known in the art. For oral or suppository formulations, 0.5-2000 mg per unit dosage, and preferably 1-1000 mg per dosage unit is taken daily.

These compositions may be used alone to treat the above-listed disorders, or alternatively, as in the case of Alzheimer's Disease, for example, they may be used as an adjunct to the conventional treatments such as haloperidol, tacrine or deprenyl.

The invention will be better understood from the Experimental Details which follow. However, one skilled in the art will readily appreciate that the specific methods and results discussed are merely illustrative of the invention as described more fully in the claims which follow thereafter.

### Introduction

Compounds of general formula I may be prepared (as shown in Scheme I) from the corresponding carbamoyl derivatives of phenethylamine IV by reacting the latter with propargyl compounds bearing an appropriate leaving group at the 3-position, e.g. a halide group, mesylate, tosylate, etc., under basic conditions provided by an inorganic base, e.g. K₂CO₃, NaOH, or an organic base e.g. a tertiary amine, in a polar organic solvent, e.g. CH₃CN, DMF, etc., at 15-40°C , preferably at 20-25°C, for a period of time in the range of 5-24 hours, preferably 5-10 hours. The products, obtained after a suitable work-up and purification, are in the form of free bases. Preferably these are converted into their pharmaceutically acceptable salts.

Compounds of general formula IV may be prepared by Boc deprotection of compounds of general formula III. Preferably the deprotected compounds obtained are converted into their hydrochloride salts. Compounds of general formula III may be prepared by carbamylating a compound of general formula II in a conventional manner, e.g. by reacting the compound of formula II with an appropriate carbamoyl halogenide or an alkylisocyanate.

Compounds of general formula II may be prepared by Boc protection of the appropriate hydroxy phenethylamines, by methods known to those skilled in the art.

Compounds of general formula I, where R₁ is not H , may also be prepared (as shown in scheme II) by carbamylation of VII, by the same method described above for the carbamylation of II. Compounds of general formula VII may be prepared by propargylation of the 3-hydroxy phenethylamines of general formula VI, by methods analogous to those described for the propargylation of IV.

N,N dialkyl substituted compounds of formula I may be prepared by the carbamylation of compounds of formula V or the N-alkylation of compounds of formula IV.

### STARTING MATERIALS

3-Hydroxy-phenethylamine and its N-methyl analogue were obtained by demethylation of 3-methoxy phenethylamine and N-methyl-3-methoxy phenethylamine. The latter was prepared from 3-methoxy phenethylamine by reductive alkylation (ethyl formate, followed by lithium aluminium hydride).

N,N-Dimethyl-3-hydroxy phenethylamine was prepared by reductive amination (H2,Pd/C,Me₂NH) of (3-methoxyphenyl) acetonitrile¹, followed by O-demethylation.

3-Hydroxy-methyl phenethylamine was obtained by O-demethylation of the corresponding 3-methoxy analogue. The latter may , be prepared by reductive amination (NaCNBH₃,NH₄OAc)² of 3-methoxyphenyl acetone³ or by reduction of 1-(3-methoxyphenyl)-2-nitro-1-propene (obtained by condensing 3-methoxy benzaldehyde with nitroethane)⁴. 3-Hydroxy-,N-dimethyl phenethylamine was prepared by O-demethylation of the corresponding 3-methoxy analogue. The latter was obtained by the reductive amination (methylamine, NaCNBH₃)² of 3-methoxyphenyl acetone³.

Alternatively, 3-hydroxy-(α,N-dimethyl phenethylamine may be prepared by reductive methylation (N-formylation followed by reduction) of 3-hydroxy-α-methyl phenethylamine.

### References:

1. JS Buck et al., *J. Amer. Chem. Soc.* (1938) 60: 1789
2. RF Borch et al., *J. Amer. Chem. Soc.* (1971) 93: 2897
3. Org. Synth. Coll. Vol IV, (1963) 573; and
4. A Carlsson, et al., *Acta. Pharm. Suecica.* (1970) 7: 293.

### Preparation of Compounds of the Present Invention

### A. Boc - protection and carbamylation

### 1. Boc protection

### ( 3-hydroxy-N-Boc,N-methyl phenethylamine)

To a solution of 3-hydroxy N-methyl phenethylamine (8.33 g, 55.17 mmol) in dioxane (80 ml) and water (80 ml) was added NaHCO₃ (13.65 g) and di-t-butyl dicarbonate (13.65 g, 62.54 mmol). The reaction mixture was stirred at room temperature (RT) for 4 hrs and evaporated to dryness in-vacuo. The residue was taken up in a water:dioxane mixture (400 ml, 1:1), and the layers were separated. The aqueous layer was re-extracted with ether (2x75 ml) and the combined ether layer was dried (Na₂SO₄) and evaporated to dryness in-vacuo. The oily residue was purified by column chromatography (hexane:EtOAc 2:1) to give 10.2 g (74%) of the title compound as a viscous yellow oil.

### 2. Carbamylation

### 3-(N-Me,N-nPr carbamyloxy) N-Boc,N-methyl phenethylamine

To an ice-cooled solution of 3-hydroxy-N-Boc,N-methyl phenethylamine (5.0 g, 19.9 mmol) in dry acetonitrile (65 ml) was added under nitrogen, N-methyl,N-n-propyl carbamoyl chloride (4.66 g, 34.43 mmol), followed by the portionwise addition of NaH (60% disp. in oil, 1.03 g, 25.87 mmol). The reaction mixture was stirred at RT under nitrogen for 6 hrs and evaporated to dryness in-vacuo. Water (200 ml) was added, the pH was adjusted to ∼9 and the aqueous layer was extracted with ether (4x100 ml). The combined ether layer was washed with NaOH solution (pH 9.5), water (150 ml), dried (Na₂SO₄) and evaporated to dryness in-vacuo to give an oil which was purified by column chromatography (hexane:EtOAc 2:1), affording 6.0 g (86%) of the title compound as a yellow oil.

According to these methods the exemplary intermediates in Table 1 were prepared.

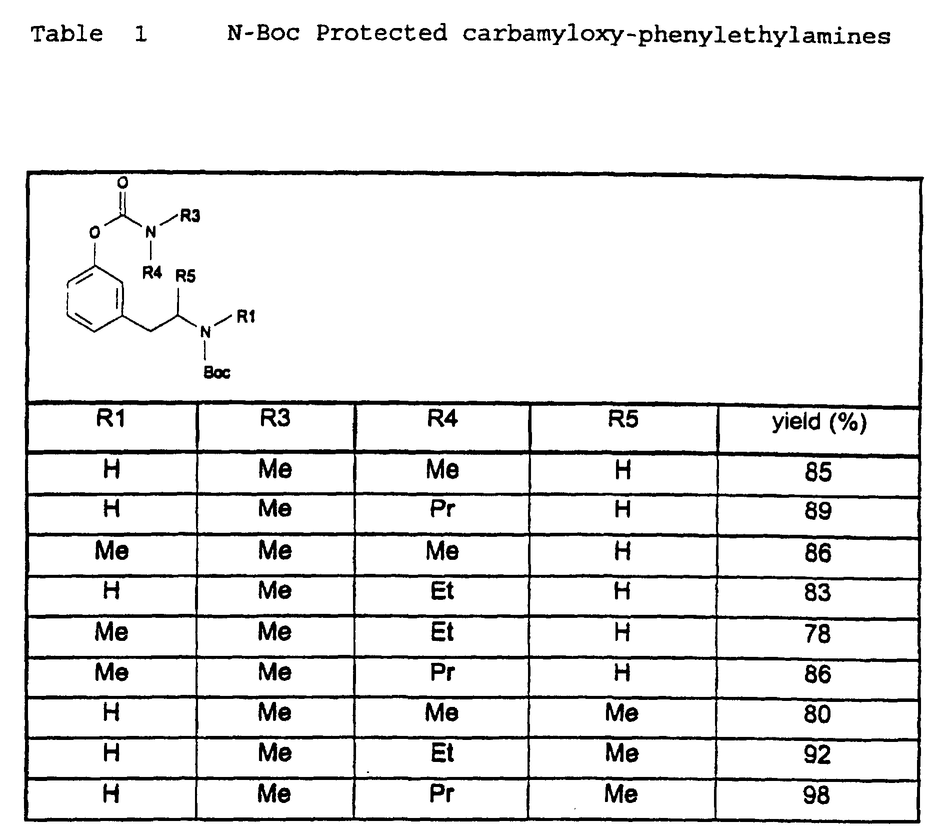

### B. Boc - Deprotection and Salt Formation

### 3-(N-Me,N-nPr Carbamyloxy) N-methyl phenethylamine . HCl (Compound 6)

3-(N-Me,N-nPr Carbamyloxy) N-Boc,N-methyl phenethylamine (6.0 g, 17.14 mmol) was dissolved in dioxane (60 ml) and 20% HCl/ether (60 ml) was added. The mixture was stirred at RT for 4 hrs and evaporated to dryness in-vacuo, and the residual oil was treated with ether (2x150 ml), to give, after stirring and ice-cooling, 4.6 g (93.5%) of the title compound as a white solid. In this manner the compounds shown in Table 2 were prepared, their analytical characteristics are given in Table 4.

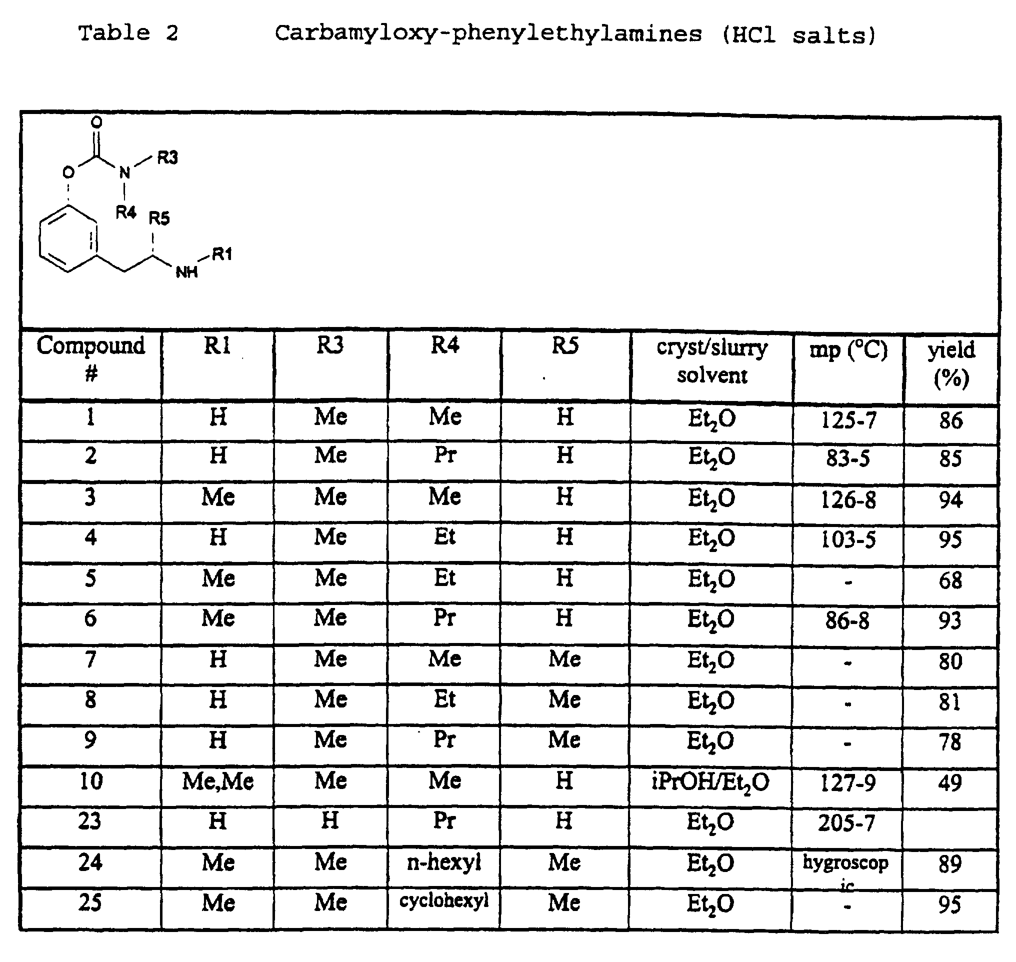

### C. Propargylation and salt formation

### 3-(N-Me,N-Et carbamyloxy)α-methyl, N-propargyl phenethylamine.HCl (Compound 17)

A solution of propargyl bromide (1.1 g, 9.1 mmol) in acetonitrile (8.5 ml) was added dropwise to a stirred mixture of 3-(N-Me, N-Et carbamyloxy)α-methyl phenethylamine.HCl (2.4 g, 8.8 mmol) and potassium carbonate (2.8 g) in acetonitrile (25 ml), and the mixture was stirred at RT for 7 hrs. The reaction mixture was filtered and the filtrate was removed under reduced pressure and the residue was purified by column chromatography (hexane:EtOAc 2:1) to give 1.76 g of the title compound as the free base (73%).

The free base was dissolved in dry ether (50 ml) and HCl/ether was added (to pH 1). The mixture was stirred for 4 hrs at RT, filtered and the solid was washed with cold ether, to give, after drying at 60°C in-vacuo, 1.5 g (4.82 mmol, 55%).

### 3-(N-Me,N-nPr carbamyloxy)N-methyl,N-propargyl phenethylamine.HCl (Compound 18)

To a stirred mixture of 3-(N-Me,N-nPr carbamyloxy)N-methyl phenethylamine.HCl (4.02 g, 14.0 mmol) and potassium carbonate (3.87g, 28.0 mmol) in acetonitrile (150 ml), was added dropwise at RT a solution of propargyl bromide (1.67 g, 14.0 mmol) in acetonitrile (10 ml). The reaction mixture was stirred at RT for 21 hrs, filtered and the filtrate evaporated to dryness in-vacuo and the residual orange oil (4.1 g) was purified by column chromatography (EtOAc) to give 2.7 g of the free base.

The free base (1.35 g, 4.69 mmol) was dissolved in dry ether (70 ml) and HCl/ether (7 ml) was added dropwise. The mixture was stirred at RT for 1/2 hr and the supernatant was decanted off. The gummy residue was crystallized twice from iPrOH/ether to give 1.16 g (51.4%) of a white solid.

The methods were repeated and the following compounds of the present invention were prepared (Table 3), their analytical characteristics are given in Table 5.

### D. Propargylation of 3-hydroxy phenethylamines

### (S)-3-Hydroxy-α,N-dimethyl-N-propargyl-phenethylamine (Compound 42)

To a solution of (S)-3-Hydroxy-α,N-dimethyl phenethylamine.HCl (4.4g, 21.8mmol) in dimethylacetamide (200 ml) stirred at 25°C under a nitrogen atmosphere, was added K₂CO₃ (6.04g, 43.64 mmol), and the mixture was stirred for 10 minutes. Then a solution of propargyl bromide (2.34g, 19.64 mmol) in dimethylacetamide (10ml) was added over 2 minutes and the mixture was stirred at 25°C for 24 hours.

Water (250ml) was added and the mixture was stirred until all the solid material dissolved. The aqueous layer was extracted with toluene (10 x 75ml). The layers were separated and the combined toluene layer was washed with saturated brine (2 x 150ml) and dried (Na₂SO₄). Removal of solvent at reduced pressure gave an orange oil, which was purified by flash column chromatography using ethyl acetate as the eluent. This gave 3.60g (90.2%) of Compound 42 as an orange oil.

Using this procedure, the (R) isomer (Compound 41) was obtained in 80%.

### E. Carbamylation and salt formation

### (S) 3-(N-methyl,N-ethyl-carbamyloxy)-(α,N-dimethyl-propargyl-phenethylamine esylate (Compound 39)

To a solution of compound 42 (1.80 g, 8.87 mmol) in dry acetonitrile (100 ml) cooled in an ice bath, was added under nitrogen N-methyl-N-cyclohexylcarbamyl chloride (2.70 g, 15.39 mmol) followed by the portionwise addition of NaH (60% oil dispersion, 0.467 g, 11.69 mmol). The mixture was then stirred at RT under nitrogen for 18 hours. Solvent was removed at reduced pressure and water (100 ml) and ether (150 ml) were added. The mixture was stirred until all the material dissolved. The layers were separated and the aqueous layer was reextracted with ether (4 x 70ml). The combined ether layers were washed with KOH solution (pH 9.5), water and dried (Na₂SO₄).

Removal of solvent at reduced pressure gave 3.87 g of an orange oil which was purified by flash column chromatography using the ethyl acetate as the eluent. This gave 2.57 g (84.5%) of the title compound (free base) as a yellow oil.

The free base was dissolved in dry EtOAc (9 ml) and a solution of 95% ethanesulfonic acid (0.79 g, 6.81 mmol) in EtOAc (1.5 ml) was added. The solution was cooled to 5°C and stirred at this temperature. After about 15 minutes, a white solid precipitated and it was stirred at 5°C for 3 hours. The solid was filtered using a minimum amount of ice-cold ethyl acetate. This gave 2.3g (75%) of a white solid having a melting point of 118-120°C.

The methods were repeated and the following compounds of the present invention were prepared (Table 3a), their analytical characteristics are given in Table 5.

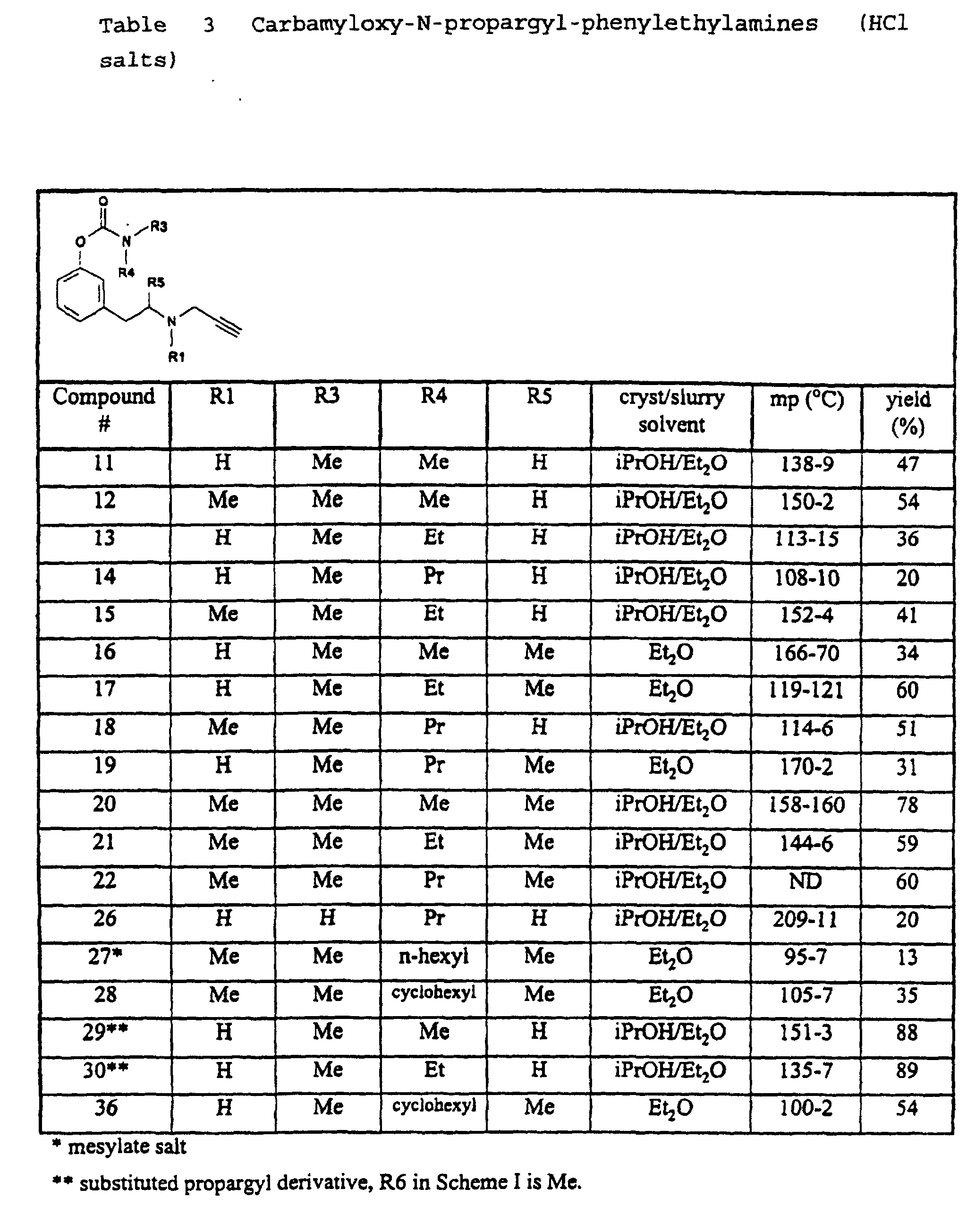

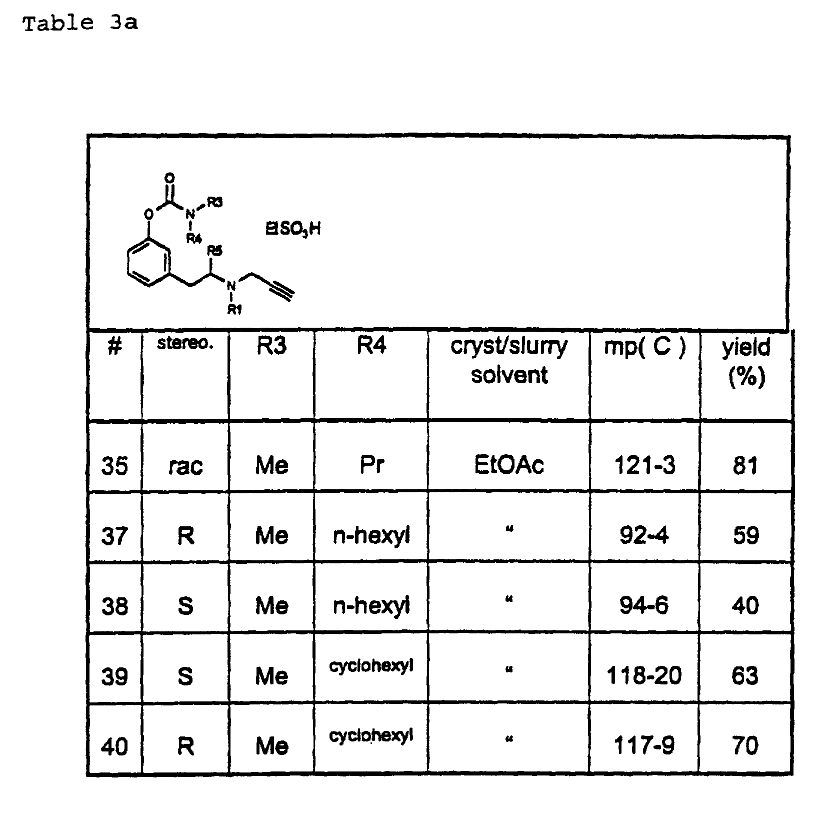

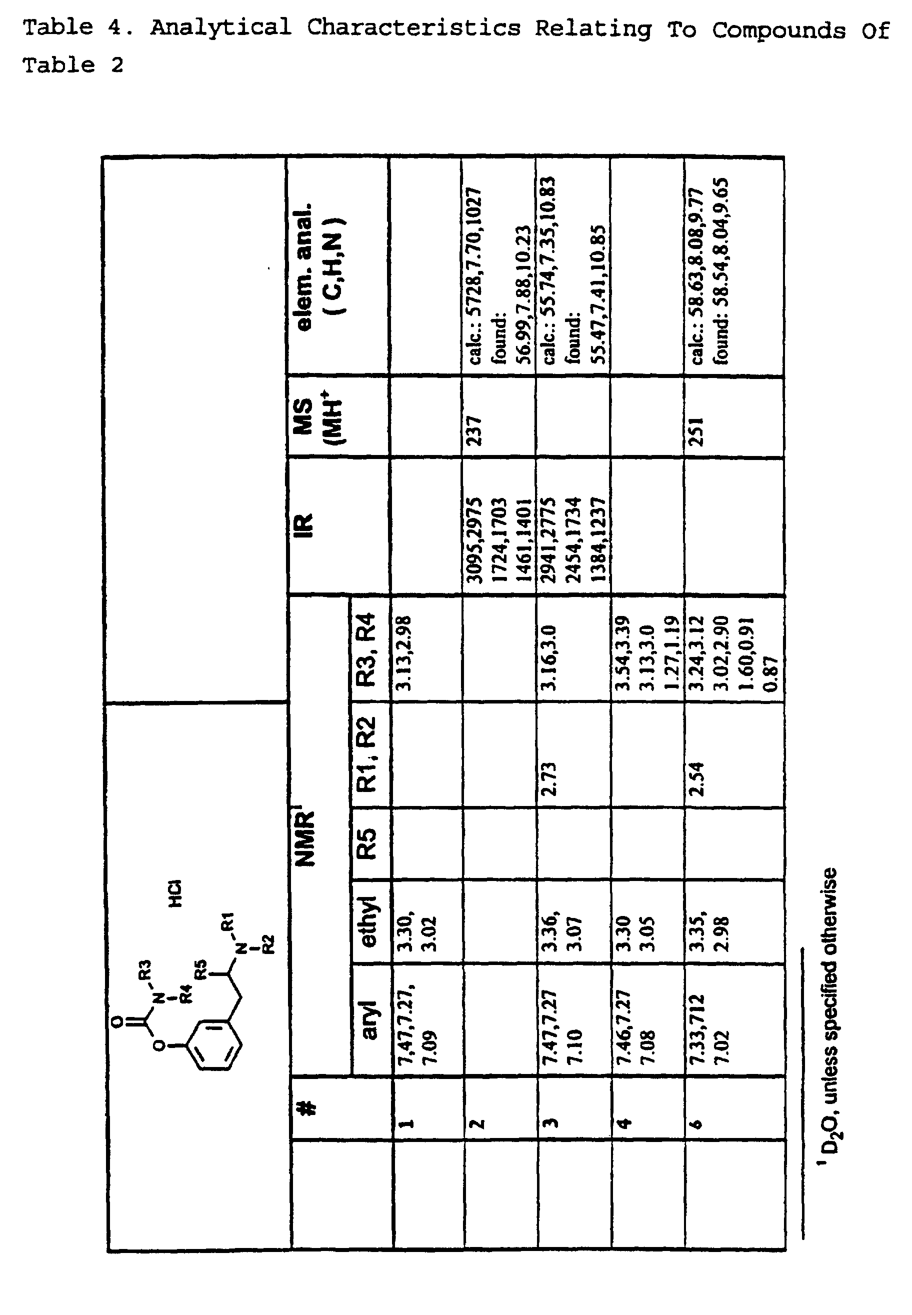

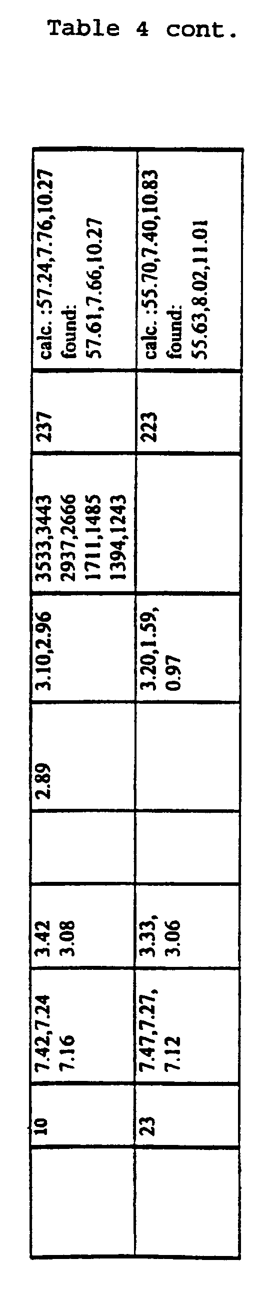

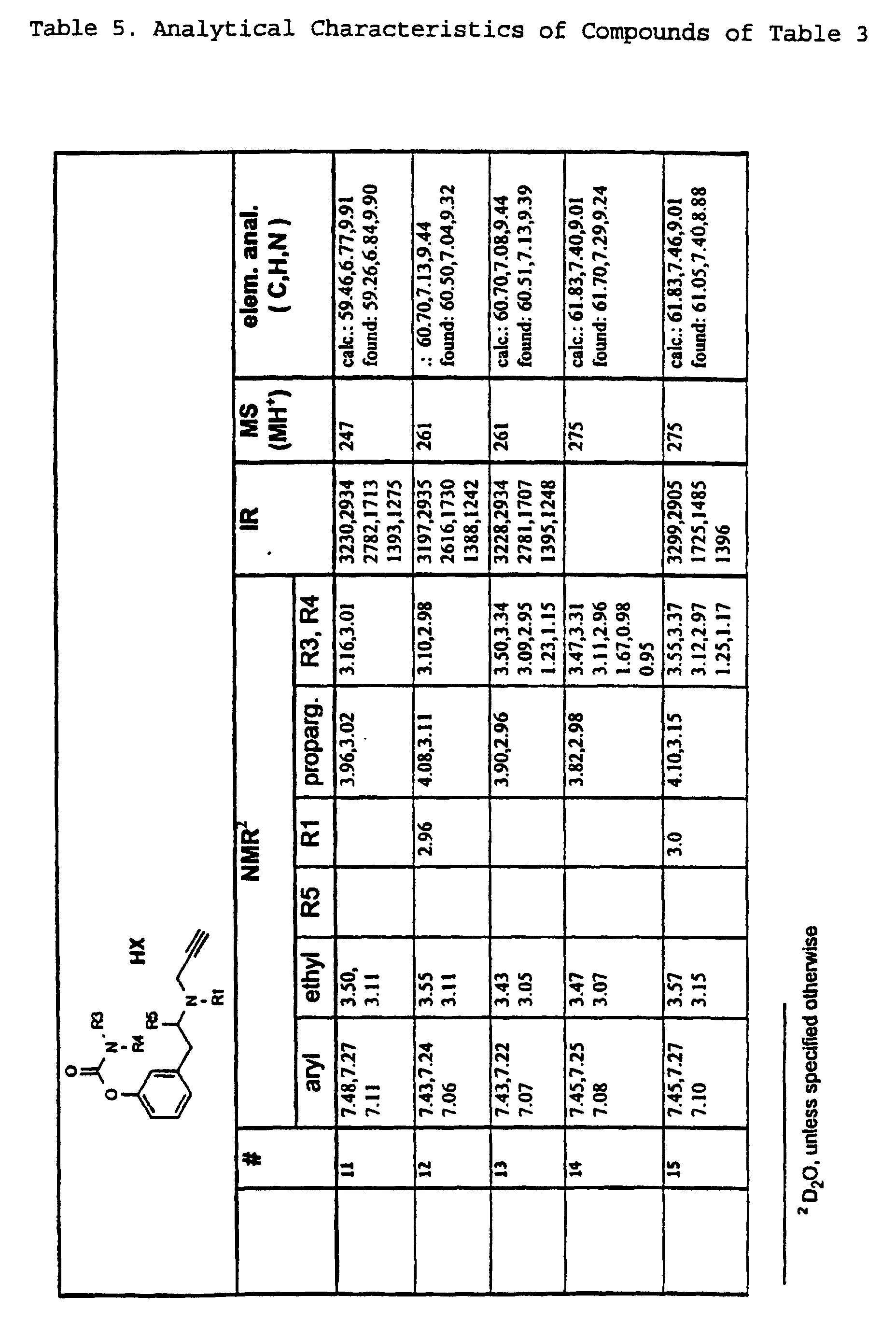

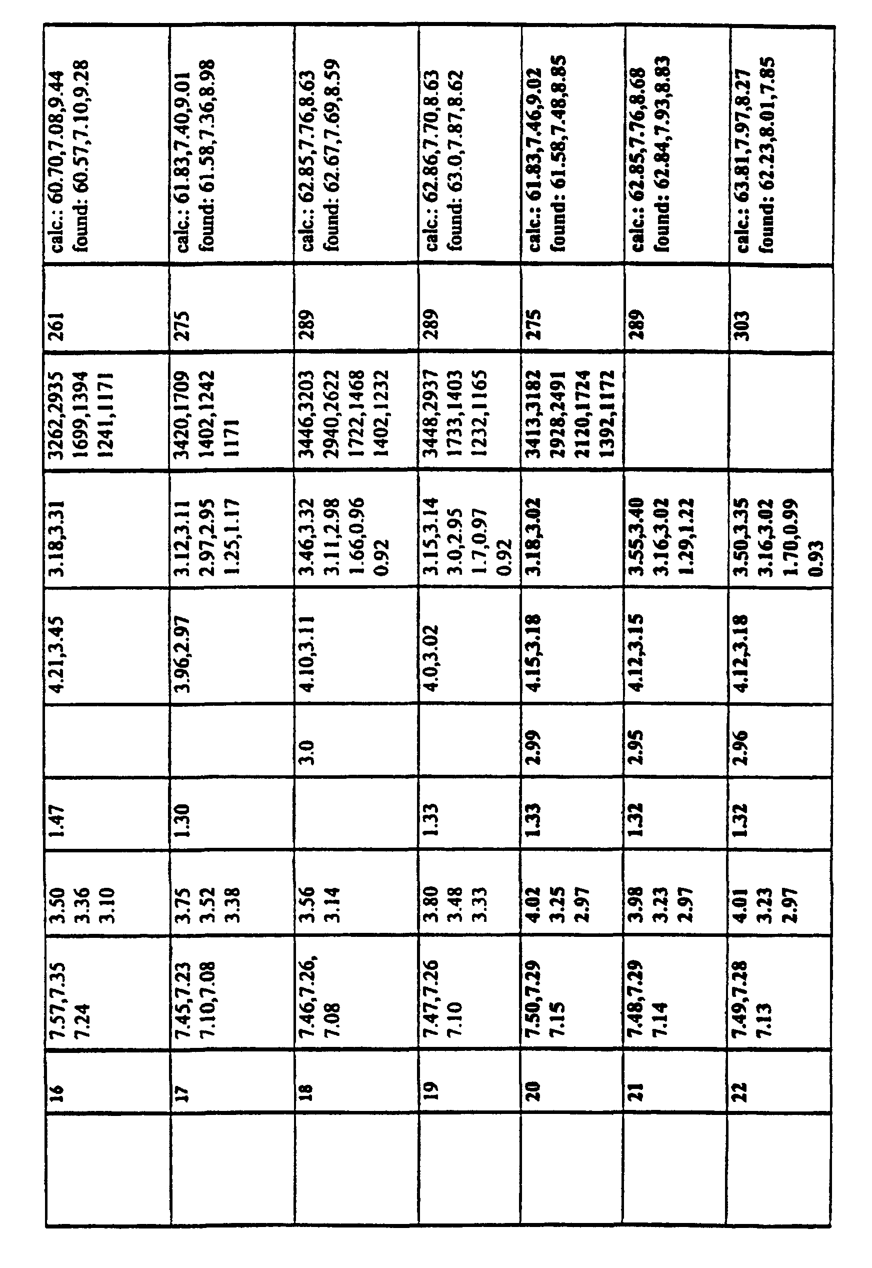

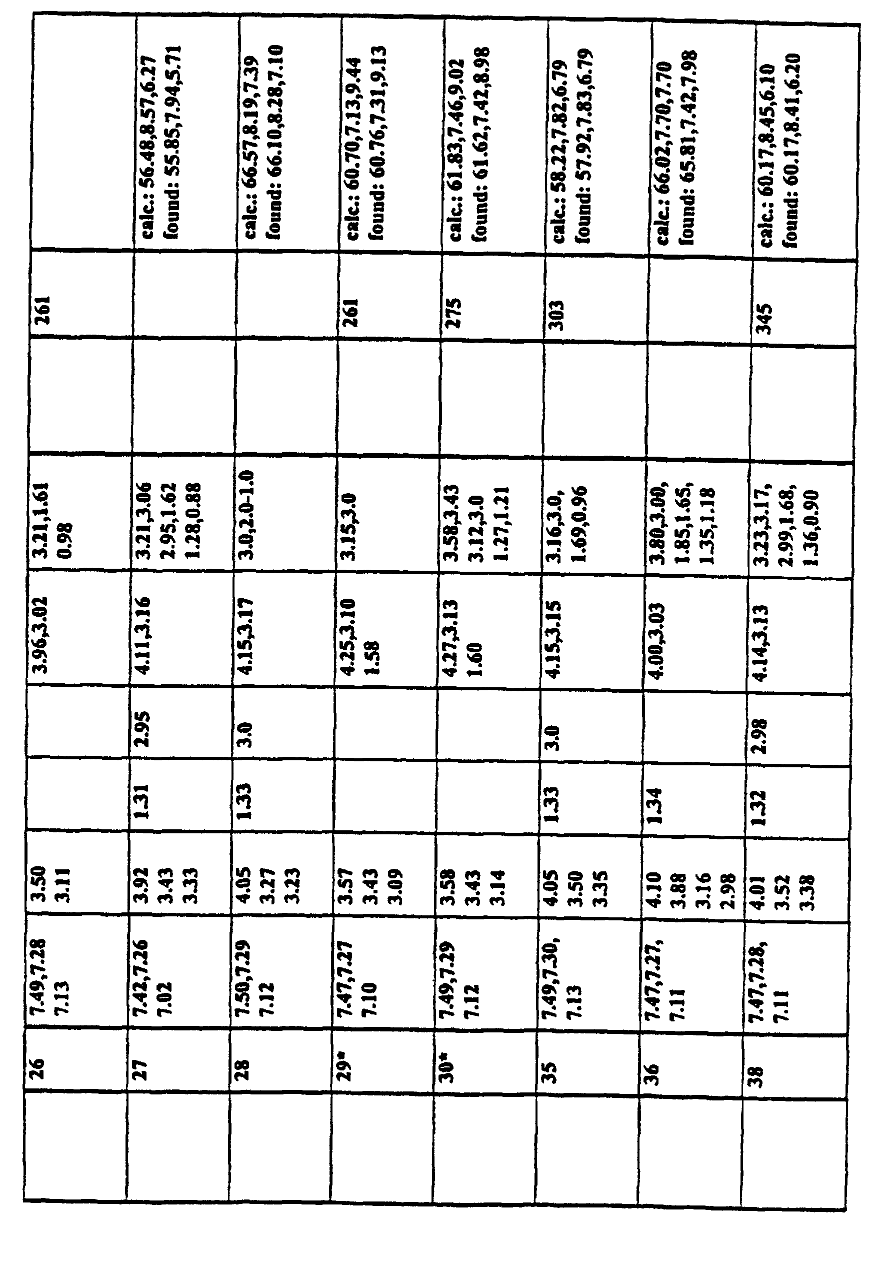

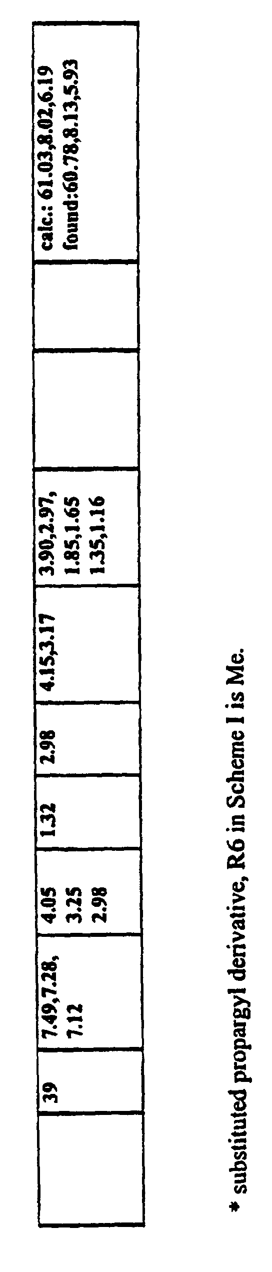

### BIOLOGICAL EXAMPLES

### Example 1

### Acetylcholinesterase Inhibition in Mice

### 1.1 In vitro measurement of Acetylcholinesterase (AChE) Inhibition

Human erythrocyte acetylcholinesterase (type XIII, Sigma Israel), was prepared in a stock solution of 1U/ml, containing Triton (1%) and bovine serum albumin (0.05%) in phosphate buffer (pH 8). The enzyme (0.05U) was incubated with 3-5 different concentrations of test compound (in triplicate) for periods of from 15 to 60 minutes at 37°C. The substrate acetylthiocholine (0.075M) and 5,5'-dithiobis-(2-nitrobenzoic acid) (DTNB, 0.01M) were then added and the rate of hydrolysis of the substrate which yields a yellow product monitored spectrophotomerically at 412nM (Ellman, et al., *Biochem Pharmacol*. (1961) 7: 88-95). The percentage inhibition of AChE by each concentration of drug is calculated by comparison with that of enzyme in the absence of drug. The concentration of each drug that inhibits AChE by 50% (IC₅₀) at the time of peak activity was calculated and is given in Table 6 below.

### 1.2 Ex vivo measurement of Acetylcholinesterase (AChE) Inhibition

Test drugs or saline were administered sub-cutaneously to male mice (Sabra strain, 28-35g). At least 4-5 mice were used per dose and a minimum of 3 doses per drug were tested. The mice were sacrificed 15, 30, 60, 70, 90, 120 or 180 minutes after drug administration, the brains rapidly removed (minus cerebellum), weighed and homogenized in 0.1M phosphate buffer pH 8.0, containing Triton (1mg/100g tissue) and centrifuged to remove cell debris. Aliquots (25 µl) of the supernatant were then incubated with acetylthiocholine and DTNB. AChE activity measured as described above. The % inhibition of whole brain AChE by each dose of drug was calculated by comparison with enzyme activity from 3 saline treated control mice run at the same time. The dose of each drug that inhibits AChE by 50% at the peak of activity (ED₅₀) was calculated and is given in Table 6.

### 1.3 Acute Toxicity in Mice

Drugs were administered sub-cutaneously in at least 3 doses, to a minimum of 10 mice per dose. The dose that was lethal to 50% of the mice (LD₅₀) within 6 hours after administration. was calculated for each drug and is given in Table 6. Therapeutic Ratio was calculated as LD₅₀ divided by ED₅₀(acetylcholine esterase ex vivo).

### Example 2

### 2.1 Inhibition of MAO activity in vitro

The MAO enzyme source was a homogenate of rat brain in 0.3M sucrose, which was centrifuged at 600G for 15 minutes. The supernatant was diluted appropriately in 0.05M phosphate buffer, and pre-incubated with serial dilutions of test compounds for 20 minutes at 37°C. ¹⁴C-Labeled substrates (2-phenylethylamine, hereinafter PEA; 5-hydroxytryptamine, hereinafter 5-HT) were then added, and the incubation continued for a further 20 minutes (PEA), or 30-45 minutes (5-HT). Substrate concentrations used were 50µM (PEA) and 1mM (5-HT). In the case of PEA, enzyme concentration was chosen so that not more than 10% of the substrate was metabolized during the course of the reaction. The deaminated products were extracted into toluene-ethyl acetate (1:1, v/v) containing 0.6% (w/v) 2,5-diphenyloxazole prior to determination by liquid scintillation counting. Radioactivity in the eluate indicates the production of neutral and acidic metabolites formed as a result of MAO activity. Activity of MAO in the sample was expressed as a percentage of control activity in the absence of inhibitors after subtraction of appropriate blank values The activity determined using PEA as substrate is referred to as MAO-B, and that determined using 5-HT as MAO-A.

Concentrations of inhibitor producing 50% inhibition of substrate metabolism (IC₅₀) were calculated from the inhibition curves, and are shown in Table 6.

### 2.2 Inhibition of MAO activity ex vivo

Male Sabra mice, weighing 45-50g were injected with test compound solutions (prepared in 0.9% saline). Each dose was administered to two or three mice. The mice were sacrificed two hours after drug administration or at a time corresponding to the peak AChE inhibition time (see Table 6). The brain and liver were rapidly dissected and stored in appropriate vials on ice. The tissues were weighed, diluted to 1/20 in sucrose 0.3M and stored at -20°C before performance of the MAO assay described above. The results given in Table 6 relate to measurements made on brain tissue only.

### 2.3 Inhibition of MAO activity following sub-acute administration to rats

Experiments were done with Spague Dawley male rats. Procedures were repeated as described in Examples 2.1 and 2.2, but drug administration was continued daily for 14 days. At the end of this period animals were sacrificed and MAO levels determined in the brain, liver and intestines. Compound 17 was administered sub-cutaneously or per os at a dose of 20mg/kg. The results are shown in Table 6a.

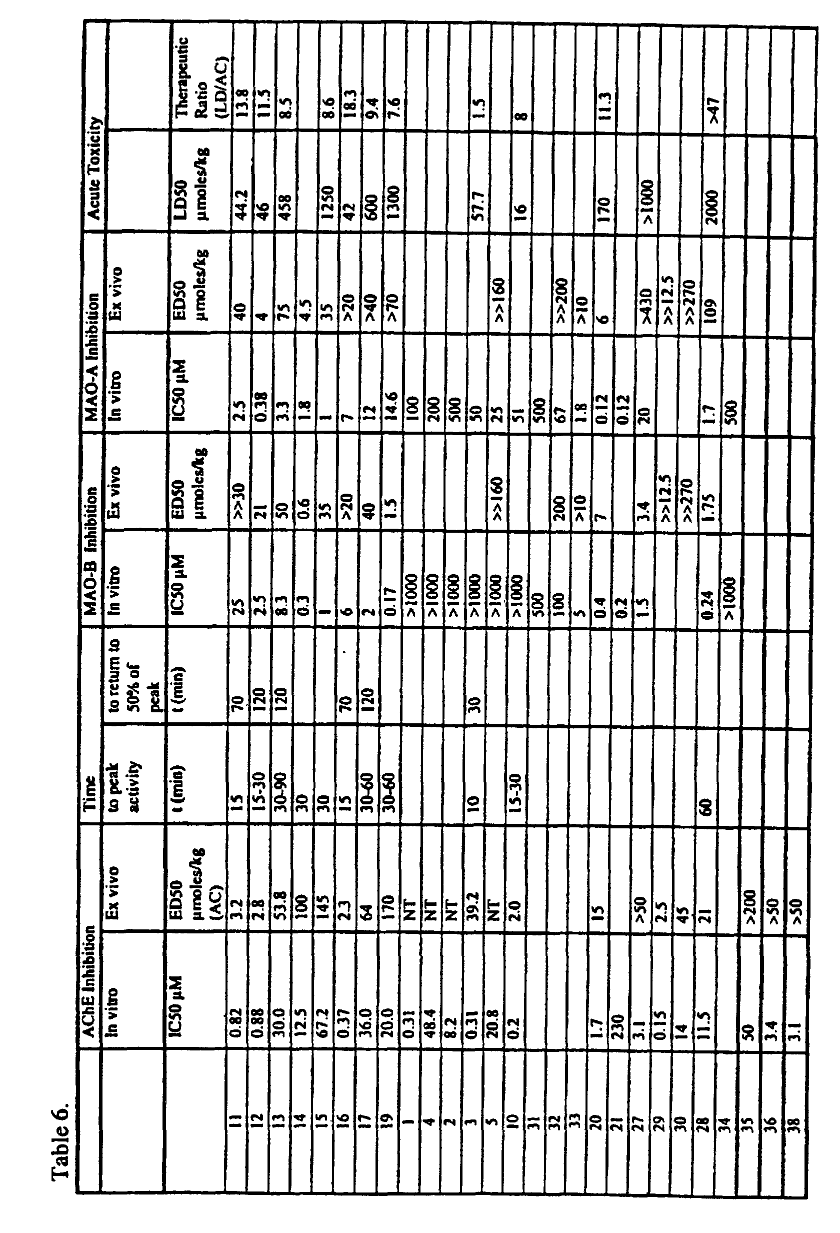

**Table 6a.**

| Effect of compound 17 on MAO activity following sub-acutechronic treatment | | | |
|---|---|---|---|
| Organ | Administration | %MAO-A inhibition | % MAO-B inhibition |
| Brain | sc | 76 | 87 |
| | po | 59 | 72 |
| Intestine | sc | 10 | 33 |
| | po | 25 | 40 |
| Liver | sc | 33 | 51 |
| | po | 59 | 74 |

### Example 3

### Effect of drug treatment following closed head injury (CHI) in mice

The procedure for closed head injury followed was as described for rats in Shohami et al. (J. Neurotrauma (1993) 10(2) 109-119) with changes as described.

Animals: Male Sabra mice (Hebrew University strain) weighing 34-40g were used. They were housed in groups of 10 per cage, in a 12hr:12hr light:dark cycle. Food and water were provided ad libitum.

Trauma was induced under ether anesthesia. A longitudinal incision was performed in the skin covering the skull and the skin retracted to expose the skull. The head was fixed manually at the lower plane of the impact apparatus. A weight of 333g was delivered by an electric device from a height of 3cm to the left hemisphere, 1-2mm lateral to the midline in the midcoronal plane. Test compounds were injected sub-cutaneously at a dosage corresponding to the ED₅₀ for acetylcholinesterase inhibition, once 15 min. after CHI.

### 3.1 Assessment of Motor Function.

Motor function and reflexes were evaluated in the injured mice at different times after closed head injury (CHI) using a neurological severity score (NSS) as shown in Table 7 below, which is modified from that described for rats (Shohami et al. supra.). One point was awarded for the lack of a tested reflex or for the inability to perform the tasks outline in the Table. The maximal score that can be reached at 1 hour post-CHI is 25 points and 21 at later times. The difference in NSS at 1hr and at any other time reflects the recovery, and is referred to as NSS. An NSS score of 15-19 at 1hr denotes severe injury, 11-14 moderate injury and less than 10 mild injury. The NSS recorded after treatment with test compound or control is shown in Table 8.

**Table 7**

| Neurological Severity Score for Mice After Closed Head Injury | | |
|---|---|---|
| Parameter | Points at 1 hour | Points at any other time |
| Inability to exit from a circle (30cm diameter) when left in its center | | |
| for 30min | 1 | |
| for 60 min | 1 | |
| for >60 min | 1 | 1 |
| Loss of righting reflex | | |
| for 10 second | 1 | |
| for 20 seconds | 1 | |
| for >30 seconds | 1 | 1 |
| Hemiplegia - inability of mouse to resist forced changes in position | 1 | 1 |
| Flexion of hind limb when lifted by tail | 1 | 1 |
| Inability to walk straight when placed on the floor | 1 | 1 |
| Reflexes | | |
| Pinna reflex | 1 | 1 |
| Corneal reflex | 1 | 1 |
| Startle reflex | 1 | 1 |
| Clinical grade | | |
| Loss of seeking behaviour | 1 | 1 |
| Prostration | 1 | 1 |
| Loss of reflexes | | |
| Left forelimb | 1 | 1 |
| Right forelimb | 1 | 1 |
| Left hindlimb | 1 | 1 |
| Right hindlimb | 1 | 1 |
| Functional test Failure in beam balancing task (0.5cm wide) | 1 | 1 |
| for 20 seconds | 1 | 1 |
| for 40 seconds | 1 | 1 |
| for > 60 seconds | | |
| Failure in round stick balancing task (0.5cm in diameter | | |
| for 10 seconds | 1 | 1 |
| Failure in beam walking task | | |
| 3cm wide | 1 | 1 |
| 2cm wide | 1 | 1 |
| 1cm wide | 1 | 1 |
| Maximum Points | 25 | 21 |

### Results

### Assessment of Motor Function.

**Table 8**

| Change in Neurological Severity Score after Closed Head Injury in Mice | | | | |
|---|---|---|---|---|
| Drug/dose | N | ΔNSS, 24 hr post-CHI | ΔNSS, 7 days post-CHI | ΔNSS, 14 days post-CHI |
| Saline, 1ml/kg | 51 | 4.75±0.17 | 5.83±0.36 | 5.96±0.4 |
| 17 | 12 | 5.58±0.38* | 6.92±0.36* | 7.83±0.53* |

| | | | | |
|---|---|---|---|---|
| * significantly different from saline control (p<0.05) | | | | |

### 3.2 Assessment of Reference Memory

Morris Water Maze Teat: the water maze consists of a circular aluminium pool, 1m in diameter and 60cm in depth, filled with water to a depth of 17.5cm. The hidden goal platform is a glass vessel (15cm diameter x 16. 5cm height) placed upside down at a fixed location in the pool, 1cm below the surface of the water. The water temperature is maintained at 24°C and the pool is always placed in the same position in the room to provide the same extra-maze cues. Prior to CHI (as described in Example 3 above), mice were given 3 trials per day for 5 consecutive days to establish a baseline performance - measured as the latency to find the platform from the same start location. Commencing 24hr after CHI, mice were retested daily for 2 weeks in 3 trials per day.

Figure 1 shows the reduction in latency for mice treated with compound 17 compared to saline treated controls after CHI. It appears that immediately post-CHI mice forget the location of the goal. Memory is enhanced following treatment with test compounds, as compared to saline created mice. In Figure 1 the arrow shows the time of CHI.

### Example 5:

### Effect On Mice Having Experienced A Hypobaric Hypoxic Episode

The hypobaric hypoxic model is a well accepted model for assessing the activity of compounds believed to possess neuroprotective activity. The model is based on that described in Nakanishi, M. et al. *Life Sci*. (1973) 13: 467; Oshiro, et al., *J. Med. Chem.* (1991) 34: 2004-2013; and US Patent 4,788,130.

A 12 liter desiccator (desiccator A) and a 2.5 liter desiccator (desiccator B) were separately connected to a vacuum pump. Desiccator B was disconnected and allowed to equilibrate with room air whilst desiccator A was evacuated to a pressure of 100mmHg. Four male ICR albino mice (22-28g) were placed in desiccator B. Desiccator B was then closed to room air and connected to desiccator A. The pressure inside desiccator B was monitored using a mercury manometer and at the point the pressure in desiccator B reached 200mmHg (usually within 14 seconds), the two desiccators were disconnected from the vacuum pump and the pump switched off. The survival time from the moment of induction of hypoxia to the time of cessation of respiration was recorded. for each mouse for a maximum of 15 minutes after which time room air was reintroduced to desiccator B. Survivors were monitored for signs of lethargy or vitality.

Effect of drug treatment was assessed as the percent of the survival time of the drug treated group with respect to the saline injected or vehicle injected control group. Control groups were run twice, before and after each experimental group and consisted of 8 mice in groups of 4 mice to ensure a constant residual volume of oxygen in all tests. The effect of each dose of test drug was determined in duplicate i.e. two groups of 4 mice. The range of survival times of control mice was from 108-180 seconds.

Positive reference drugs were sodium pentobarbital at a dose of 40 mg/kg, and diazepam 10 mg/kg given 0.5h prior to hypoxia, physostigmine 0.2 and 0.4 mg/kg and neostigmine 0.2 mg/kg given sc 30 min before hypoxia. Methyl atropine 1 mg/kg was given sc. 10 min. before physostigmine.

Test drugs were dissolved in 0.9% saline, and injected sc. in the nip of the neck at a dose in accordance with body weight, 60-90 min. before hypoxia. The volume of injection was 0.2-0.3 mL per mouse (10 mL/kg). The initial dose was about one third of the reported LD₅₀ for acetylcholine esterase inhibition. If no protection could be obtained, the dose was further increased to the nearest non-toxic dose. In case of protection, the dose was further reduced in an attempt to locate the "protective" dose range.

Per cent survival times as compared to saline treated control is shown in Table 9.

**Table 9.**

| Survival Time of Mice Having Experienced a Hypobaric Episode | | | | |
|---|---|---|---|---|
| Compound | Dose mg/kg | Time of dose ( min before hypoxia) | Protection (% of control) | p |
| Control (saline) | | | 100 | |
| Nembutal | 40 | 30 | 253±200 | <0.005 |
| Diazepam | 10 | 30 | 316±78 | <0.003 |
| Neostigmine | 0.2 | 30 | 141±32 | <0.01 |
| Physostigmine | 0.2 0.4 | 30 30 | 453±222 552±210 | <0.001 <0.001 |
| Physostigmine and Atropine methyl nitrate | 0.4 1.0 | 30 40 | 296±193 | <0.05 |
| 11 | 6.2 | 60 | 331±168 | |
| | 4.1 | 60 | 416±211 | |
| | 4.1 | 30 | 501±201 | |
| | 2 | 60 | 128±40 | |
| | 2 | 30 | 302±212 | |
| 12 | 7 | 60 | 217±120 | <0.02 |
| | 4.5 | 60 | 97±36 | NS |
| 17 | 94 | 60 | 281±158 | <0.001 |
| | 62 | 60 | 419±122 | <0.001 |
| | 31 | 60 | 149±44 | <0.05 |
| 16 | 15 | 60 | toxic | |
| | 6.2 | 60 | 324±155 | <0.001 |
| | 4.1 | 60 | 335±202 | <0.01 |
| 19 | 75 | 60 | 773±228 | p<0.001 |
| | 50 | 60 | 309±253 | p<0.05 |
| | 25 | 60 | 169±50 | p<0.01 |

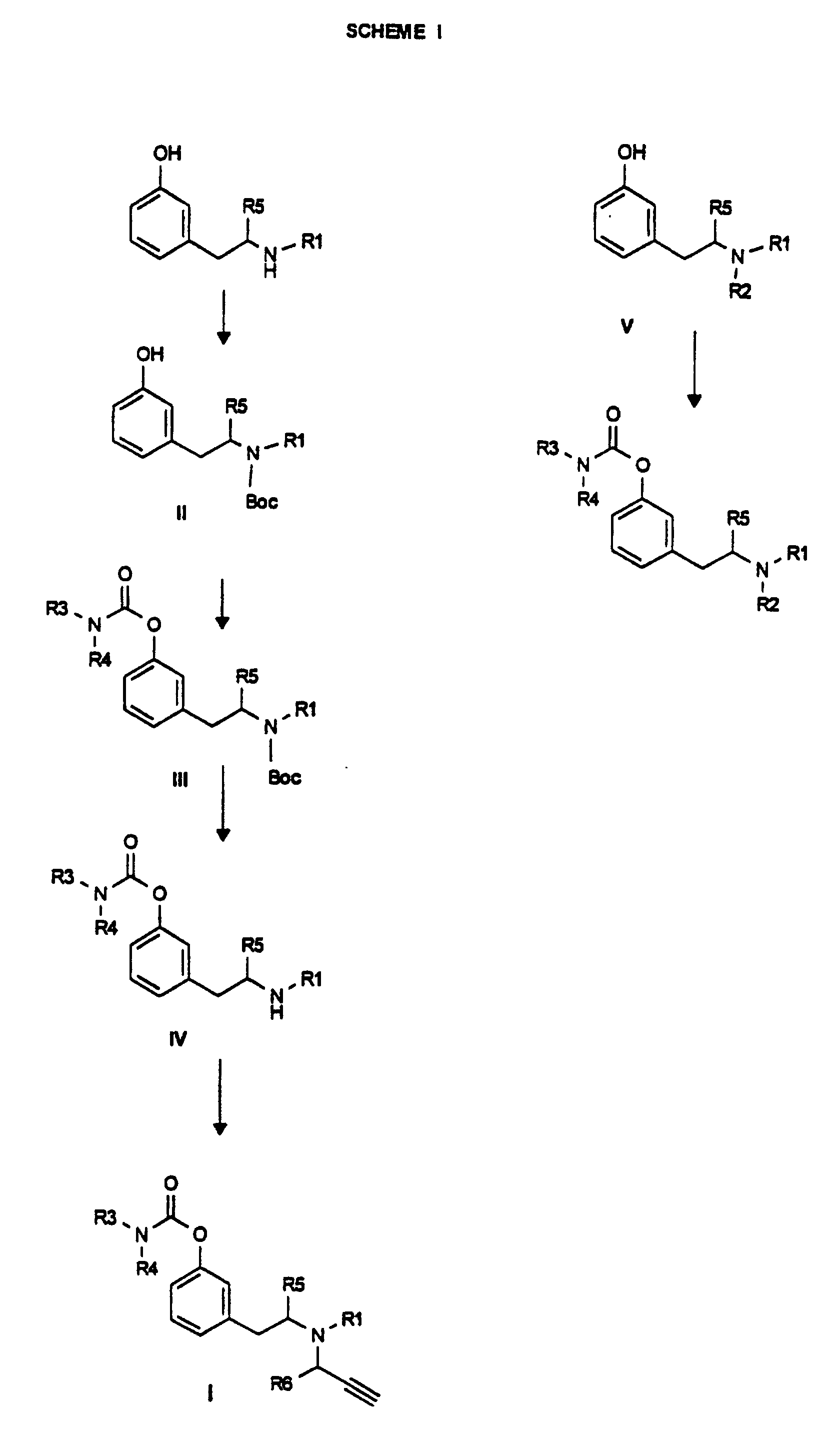

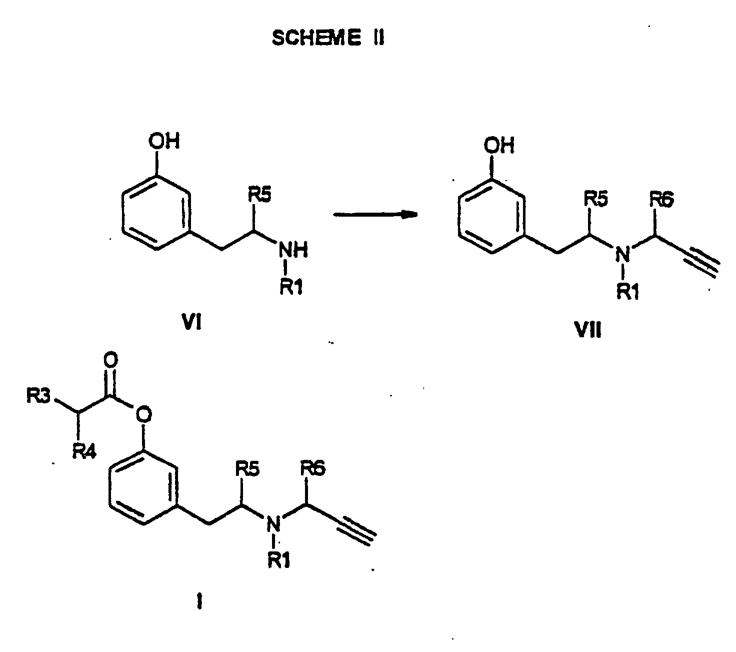

## Claims

1. A compound having the formula: 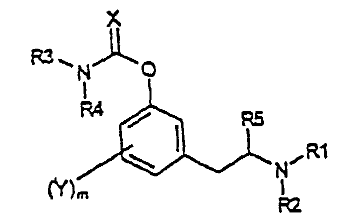 wherein m is from 0-4;
X is O or S;
Y is halogeno;
R₁ is hydrogen or C₁₋₄ alkyl;
R₂ is hydrogen, C₁₋₄ alkyl or propargyl optionally substituted by C₁₋₄ alkyl; and
R₃ and R₄ are each independently hydrogen, C₁₋₈ alkyl, C₆₋₁₂ aryl, C₆₋₁₂ cycloalkyl or C₆₋₁₂ aralkyl, R₅ is hydrogen or C₁₋₄ alkyl; and pharmaceutically acceptable salts thereof, provided that when X is O, R₂ is optionally substituted propargyl.

2. A compound according to claim 1 wherein X is O or S.

3. A compound according to claim 1, wherein the compound is selected from the group consisting of: (rac)-3-(N-methyl,N-propyl-carbamyloxy)-α-methyl-N'-propargyl phenethylamine HCl; (rac)-3-(N,N-dimethyl-carbamyloxy)-α-methyl-N'-methyl, N'-propargyl phenethylamine HCl; (rac)-3-(N-methyl,N-hexyl-carbamyloxy)-α-methyl-N'-methyl, N'-propargyl phenethylamine mesylate; (rac)-3-(N-methyl, N-cyclohexyl-carbamyloxy)-α-methyl-N'-methyl, N'-propargyl phenethylamine HCl; and (S)-3-(N-methyl, N-hexyl-carbamyloxy)-α-methyl-N'-methyl,N'-propargyl phenethylamine ethanesulfonate.

4. A compound according to claims 1 or 2, wherein at least one of R₃ and R₄ is methyl and the other is hydrogen, methyl, ethyl, propyl, hexyl or cyclohexyl.

5. A compound according to any of claims 1 to 4, wherein said compound is an optically active enantiomer.

6. A compound according to any of claims 1 to 5, wherein R₂ is an optionally substituted propargyl.

7. A compound according to claim 1, wherein
m is 0;
X is O;
R₁ is methyl;
R₂ is propargyl;
R₃ is methyl;
R₄ is ethyl; and
R₅ is methyl.

8. The compound of any of claims 1 to 7 for use in the treatment of neurotrauma, ADD, ADHD, Tourette's Syndrome, memory disorder or depression.

9. The compound of claim 8, wherein neurotrauma includes central nervous system damage and peripheral nervous system damage.

10. The compound of claim 9, wherein central nervous system damage and peripheral nervous system damage is selected from the group consisting of damage caused by virtue of .ischemic damage such as stroke, hypoxia or anoxia, neurodegenerative diseases, Parkinson's Disease, Alzheimer's Disease, Huntington's Disease, neurotoxic injury, head trauma injury, spinal trauma injury, and peripheral neuropathy.

11. A pharmaceutical composition comprising a therapeutically effective amount of the compound of any of claims 1 to 7 and a pharmaceutically acceptable carrier.

12. The compound of any of claims 1 to 7 for use in the treatment of Alzheimer's disease or dementias.

13. The compound of claim 12, wherein dementias include static dementia, Alzheimer's-type dementia, senile dementia, presenile dementia, progressive dementia, vascular dementia or Lewy body dementia.

14. Use of the compound of any of claims 1 to 7 for the preparation of a medicament.

## Patentansprüche

1. Verbindung der Formel: 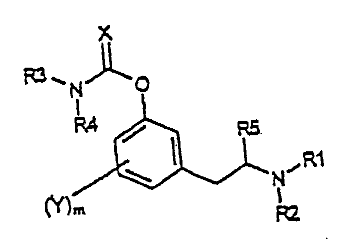 wobei m gleich 0 bis 4 ist;
X gleich O oder S ist;
Y ein Halogenatom ist;
R₁ ein Wasserstoffatom oder ein C₁₋₄-Alkylrest ist;
R₂ ein Wasserstoffatom, ein C₁₋₄-Alkylrest oder ein Propargylrest ist, gegebenenfalls substituiert mit einem C₁₋₄-Alkylrest; und
R₃ und R₄ jeweils unabhängig voneinander ein Wasserstoffatom, ein C₁₋₈-Alkyl-, C₆₋₁₂-Aryl-, C₆₋₁₂-Cycloalkylrest oder C₆₋₁₂-Aralkylrest sind, R₅ ein Wasserstoffatom oder ein C₁₋₄-Alkylrest ist; und ein pharmazeutisch verträgliches Salz davon, mit der Maßgabe, dass wenn X gleich O ist, R₂ ein gegebenenfalls substituierter Propargylrest ist.

2. Verbindung gemäß Anspruch 1, wobei X gleich O oder S ist.

3. Verbindung gemäß Anspruch 1, wobei die Verbindung ausgewählt ist aus: (rac)-3-(N-Methyl, N-propyl-carbamyloxy)-α-methyl-N'-propargylphenethylamin HCl; (rac)-3-(N,N-Dimethyl-carbamyloxy)-α-methyl-N'-methyl, N'-propargylphenethylamin HCl; (rac)-3-(N-Methyl, N-hexyl-carbamyloxy)-α-methyl-N'-methyl, N'-propargylphenethylamin Mesylat; (rac)-3-(N-Methyl, N-cyclohexyl-carbamyloxy)-α-methyl-N'-methyl, N'propargylphenethylamin HCl und (S)-3-( N-Methyl, N-hexyl-carbamyloxy)-α-methyl-N'-methyl, N'-propargylphenethylamin Ethansulfonat.

4. Verbindung gemäß Anspruch 1 oder 2, wobei mindestens ein Rest von R₃ und R₄ eine Methylgruppe ist und der andere ein Wasserstoffatom, eine Methyl-, Ethyl-, Propyl-, Hexyl- oder Cyclohexylgruppe ist.

5. Verbindung gemäß einem der Ansprüche 1 bis 4, wobei die Verbindung ein optisch aktives Enantiomer ist.

6. Verbindung gemäß einem der Ansprüche 1 bis 5, wobei R₂ ein gegebenenfalls substituierter Propargylrest ist.

7. Verbindung gemäß Anspruch 1, wobei
m gleich 0 ist;
X gleich O ist;
R₁ eine Methylgruppe ist;
R₂ eine Propargylgruppe ist;
R₃ eine Methylgruppe ist;
R₄ eine Ethylgruppe ist; und
R₅ eine Methylgruppe ist.

8. Verbindung gemäß einem der Ansprüche 1 bis 7 zur Verwendung in der Behandlung eines Nerventraumas, ADD, ADHD, des Tourette-Syndroms, eines Gedächtnisleidens oder Depression.

9. Verbindung gemäß Anspruch 8, wobei das Nerventrauma Schäden des zentralen Nervensystems und des peripheren Nervensystems einschließt.

10. Verbindung gemäß Anspruch 9, wobei der Schaden des zentralen Nervensystems und der Schaden des peripheren Nervensystems aus einem Schaden ausgewählt sind, welcher durch die Wirkung eines ischämischen Schadens wie Schlaganfall, Hypoxie oder Anoxie, neurodegenerativen Krankheiten, Parkinson-Krankheit, Alzheimer-Krankheit, Huntington-Krankheit, einer neurotoxischen Verletzung, einer traumatischen Kopfverletzung, einer traumatischen Wirbelsäulenverletzung und einer peripheren Neurophatie verursacht wird.

11. Arzneimittel, umfassend eine therapeutisch wirksame Menge der Verbindung gemäß einem der Ansprüche 1 bis 7 und einen pharmazeutisch verträglichen Träger.

12. Verbindung gemäß einem der Ansprüche 1 bis 7 zur Verwendung in der Behandlung von Alzheimer-Krankheit oder Demenzen.

13. Verbindung gemäß Anspruch 12, wobei die Demenzen statische Demenz, Demenz vom Alzheimer-Typ, Altersdemenz, Dementia praesenilis, progressive Demenz, vaskuläre Demenz oder Lewy-Body Demenz einschließen.

14. Verwendung der Verbindung gemäß einem der Ansprüche 1 bis 7 zur Herstellung eines Medikaments.

## Revendications

1. Composé répondant à la formule : 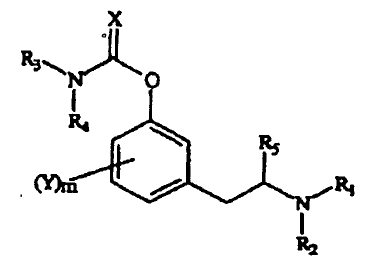 dans laquelle m vaut 0 à 4 ;
X est O ou S ;
Y est un atome d'halogène ;
R₁ est un atome d'hydrogène ou un groupe alkyle en C₁-C₄ ;
R₂ est un atome d'hydrogène, un groupe alkyle en C₁-C₄ ou un propargyle éventuellement substitué par C₁-C₄; et R₃ et R₄ sont chacun indépendamment des atomes d'hydrogène, des groupes alkyle en C₁-C₈, aryle en C₆-C₁₂, cycloalkyle en C₆-C₁₂, ou aralkyle en C₆-C₁₂, R₅ est un atome d'hydrogène ou un groupe alkyle en C₁-C₄ ; et
les sels pharmaceutiquement acceptables de ceux-ci, à condition que lorsque X est O, R₂ soit un groupe propargyle éventuellement substitué.

2. Composé selon la revendication 1, dans lequel X est O ou S.

3. Composé selon la revendication 1, dans lequel le composé est choisi dans le groupe constitué par : le chlohydrate de (rac)-3-(N-méthyl, N-propyl-carbamyloxy)-α-méthyl-N'-propargylphénéthylamine ; le chlohydrate de (rac)-3-(N,N-diméthyl-carbamyloxy)-α-méthyl-N'-méthyl, N'-propargylphénéthylamine ; le mésylate de (rac)-3-(N-méthyl, N-hexylcarbamyloxy)-α-méthyl-N'-méthyl, N'-propargylphénéthylamine ; le chlohydrate de (rac)-3-(N-méthyl,N-cyclohexylcarbamyloxy)-α-méthyl-N'-méthyl, N'-propargylphénéthylamine ; et l'éthanesulfonate de (S)-3-(N-méthyl, N-hexylcarbamyloxy)-α-méthyl-N'-méthyl, N'-propargylphénéthylamine.

4. Composé selon la revendication 1 ou 2, dans lequel l'un au moins des radicaux R₂ et R₃ est un groupe méthyle et l'autre est un atome d'hydrogène, un groupe méthyle, éthyle, propyle, hexyle ou cyclohexyle.

5. Composé selon l'une quelconque des revendications 1 à 4, dans lequel ledit composé est un énantiomère optiquement actif.

6. Composé selon l'une quelconque des revendications 1 à 5, dans lequel R₂ est un groupe propargyle éventuellement substitué.

7. Composé selon la revendication 1, dans lequel
m vaut 0 ;
X est O ;
R₁ est un groupe méthyle ;
R₂ est un groupe propargyle ;
R₃ est un groupe méthyle ;
R₄ est un groupe éthyle ; et
R₅ est un groupe méthyle.

8. Composé selon l'une quelconque des revendications 1 à 7, à utiliser dans le traitement de traumatisme neurologique, de trouble déficitaire de l'attention, de trouble déficitaire de l'attention avec hyperactivité, du syndrome de la Tourette, de trouble de la mémoire ou de dépression.

9. Composé selon la revendication 8, dans lequel le traumatisme neurologique englobe une lésion du système nerveux central et une lésion du système nerveux périphérique.

10. Composé selon la revendication 9, dans lequel la lésion du système nerveux central et la lésion du système nerveux périphérique sont choisies dans le groupe constitué par une lésion provoquée par une lésion ischémique telle qu'un accident vasculaire cérébral, l'hypoxie ou l'anoxie, les maladies neurodégénératives, la maladie de Parkinson, la maladie d'Alzheimer, la maladie d'Huntington, une lésion neurotoxique, un trauma de la tête, un trauma spinal et une neuropathie périphérique.

11. Composition pharmaceutique comprenant une quantité thérapeutiquement active du composé selon l'une quelconque des revendications 1 à 7 et un véhicule pharmaceutiquement acceptable.

12. Composé selon l'une quelconque des revendications 1 à 7, à utiliser dans le traitement de la maladie Alzheimer ou de démences.

13. Composé selon la revendication 12, dans lequel les démences englobent la démence statique, la démence de type Alzheimer, la démence sénile, la démence présénile, la démence progressive, la démence vasculaire ou la démence à corps de Lewy.

14. Utilisation du composé selon l'une quelconque des revendications 1 à 7, pour la préparation d'un médicament.
